# EUROPEAN PATENT SPECIFICATION

(11) **EP 1 121 448 B1**
(45) Date of publication and mention of the grant of the patent: **15.12.2004**
(21) Application number: 99949308.3
(22) Date of filing: 14.10.1999
(51) Int. Cl.: C12N 15/62, C12N 15/12, C12N 5/10, A61K 38/17

(54) **EXPRESSION AND SECRETION OF icIL-1 RECEPTOR ANTAGONIST TYPE II**
EXPRESSION UND SEKRETION VON icIL-1 REZEPTORANTAGONIST TYP II
EXPRESSION ET SECRETION D'UN ANTAGONISTE DE TYPE II DU RECEPTEUR icIL-1

(30) Priority: 14.10.1998 IL 12656298
(43) Date of publication of application: 08.08.2001
(73) Proprietor: INTERPHARM LABORATORIES LTD., Ness-Ziona 76110 (IL); Applied Research Systems ARS Holding N.V., Curacao (AN)
(72) Inventor: AMITAI, Hagit, 76248 Rehovot (IL); CHITLARU, Edith, 76302 Rehovot (IL)
(74) Representative: Weiss, Wolfgang, Dipl.-Chem. Dr.
(86) International application number: PCT/IL1999/000543
(87) International publication number: WO 2000/022146

(56) References cited:
- WO-A-96/12022
- MUZIO M ET AL: "CLONING AND CHARACTERIZATION OF A NEW ISOFORM OF THE INTERLEUKIN 1 RECEPTOR ANTAGONIST" JOURNAL OF EXPERIMENTAL MEDICINE,JP,TOKYO, vol. 182, no. 2, 1 August 1995 (1995-08-01), pages 623-628, XP000564500 ISSN: 0022-1007 cited in the application
- HASKILL S. ET AL.: "cDNA cloning of an intracellular form of the human interleukin-1 receptor antagonist associated with the epithelium" PROC. NATL. ACAD. SCI. USA, vol. 88, 1991, pages 3681-3685, XP002131133 cited in the application
- PECCEU F. ET AL.: "Human interleukin 1-beta fused to the human growth hormone signal peptide is N-glycosylated and secreted by Chinese hamster ovary cells." GENE, vol. 97, 1991, pages 253-258, XP002131134 cited in the application

## Description

### Field of the Invention

The present invention relates to the expression and secretion of recombinant proteins produced by DNA plasmid expression vectors in mammalian cells. More particularly this invention relates to the recombinant production of intracellular IL-1 receptor antagonist (icIL-1ra) type II by cultured COS and CHO cells, by use of DNA expression vectors containing the genomic DNA sequence of the human growth hormone (hGH) signal peptide and the cDNA of icIL-1ra type II.

### Background of the Invention

IL-1 (IL-1α and IL-1β) is a pleiotropic cytokine that exerts a variety of effects on different tissues (Dinarello, 1991 ). IL-1 affects nearly every cell type, either alone or in synergy with other cytokines (Dinarello, 1996). Two natural pathways of negative regulation strictly control the potent inflammatory effects of IL-1, under physiological conditions. One is IL-1 receptor type II, which is a non-signaling cell-surface IL-1 binding molecule, that acts as a decoy target for IL-1 (Colotta et al, 1993; Sims et al, 1993; Colotta et al, 1994). The second is the unique, IL-1 receptor antagonist (IL-1ra) (Hannum et al, 1990; Eisenberg et al, 1990; Carter et al, 1990) polypeptide that binds both surface IL-1 receptors, and inhibits signaling from the functional IL-1 receptor.

Two forms of IL-1ra have been identified. The first was a secreted form, soluble IL-1ra (sIL-1ra), that contains a classical 25-amino acid signal peptide (Eisenberg et al, 1990; Carter et al, 1990). The second, which does not contain any signal peptide, was termed intracellular IL-1ra (icIL-1ra) (Haskill et al, 1991). icIL-1ra was in fact found to be constitutively expressed intracellularly, in keratinocytes and in epithelial cells. icIL-1ra was shown to inhibit exogenous IL-1 dependent responses (Haskill et al, 1991).

The two IL-1ra isuforms are derived from the same gene. icIL-1ra transcript originates from an alternative start site, and splicing of an alternative first exon into an internal splice acceptor site located in the first exon of sIL-1ra (Haskill et al, 1991). These proteins are thus identical, except in their NH₂ end, in which the 21 amino acid signal peptide of sIL-1ra is substituted by three amino acids in icIL-1ra. sIL-1ra and icIL-1ra have a similar capability to inhibit IL-1 activity (Bertini et al, 1992) although expression of the two antagonists is differentially regulated (Haskill et al, 1991).

An additional isoform of icIL-1ra, termed the type II icIL-1ra, has been recently identified, cloned and functionally characterized (Muzio et al, 1995, WO 96/12022). The type II icIL-1ra contains an additional, in-frame, 63 bp sequence located three codons downstream of the translation start of icIL-1ra. This additional sequence is inserted between the first and the second exons of the intracellular form of IL-1ra. The additional exon is coded by an extra exon located 2kb downstream of the first icIL-1ra specific exon.

Human growth hormone (hGH) is a 191-amino acid protein synthesized and secreted by the somatotroph cells of the anterior pituitary. The hGH gene contains five exons and is the best characterized of the five members of the hGH gene family (DeNoto et al, 1981). *In vitro* transfection of the hGH gene into mammalian cells was found to yield high levels of secreted protein proportional to the levels of cytoplasmic hGH mRNA. Thus, secretion does not appear to be the rate-limiting step for appearance of hGH in the culture medium (Selden et al, 1986). The hGH gene includes a 26 amino acid signal peptide.

Pecceu et al (1991) discloses an attempt to use the human growth hormone signal peptide to create a hybrid gene with the mature form of interleukin-1β (IL-1β) in order to cause mammalian cells to secrete recombinant IL-1β. Natural IL-1β is expressed initially as an intracellular 31-kDa precursor polypeptide. When proteolytic processing of the precursor occurs, secretion of a mature 17-kDa IL-1β in a soluble mature non-glycosylated form occurs. Pecceu discloses that fusion of the mature form of IL-1β to the heterologous hGH leader sequence permitted the mature IL-1β to be secreted in mature form in CHO cells, although the form which was secreted was a glycosylated form as opposed to the non-glycosylated natural form. Pecceu discloses that the glycosylated form is biologically active. However, Pecceu further states that when the biologically active part of IL-1β was preceded only by a methionine and synthesized in CHO cells, a considerable percentage of the IL-1β produced was quite unexpectedly found in the culture medium. This disclosure leaves some amount of doubt as to whether it was the hGH signal peptide which caused the expression of the IL-1β in the CHO cells or whether such expression was specific to the mechanism involved with this particular protein, the mature form of which is naturally secreted after a precursor protein is expressed intracellularly and then cleaved to form the mature protein which is secreted. Furthermore, Pecceu reports no results as to whether the non-natural glycosylated form of IL-1β creates an immunologic reaction when administered to a human or is recognized as a self protein.

Specific situations involving the recombinant production of non-secretory proteins by fusing a signal peptide of another secretory protein are disclosed in Bjorkdahl et al (1997) and Komada et al (1997).

### Summary of the Invention

The present invention provides a method for the production of a recombinant intracellular protein, icIL-1ra type II, in mammalian cells. More particularly, the invention provides a process for engineering proteins to be secreted by use of a signal peptide derived from hGH in different expression vectors and to produce the secreted proteins in different mammalian cells.

### Description of the Figures

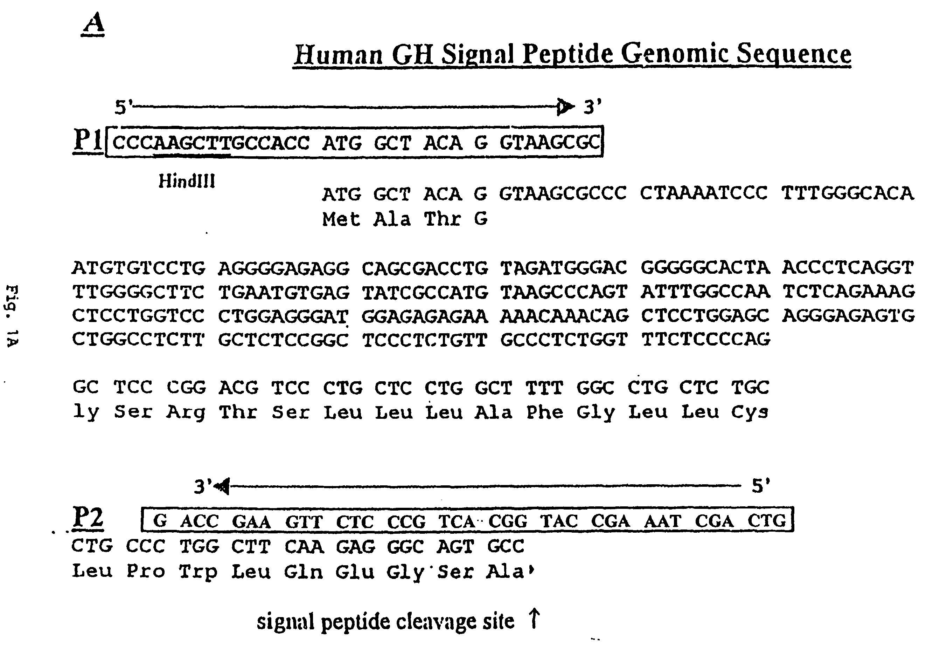

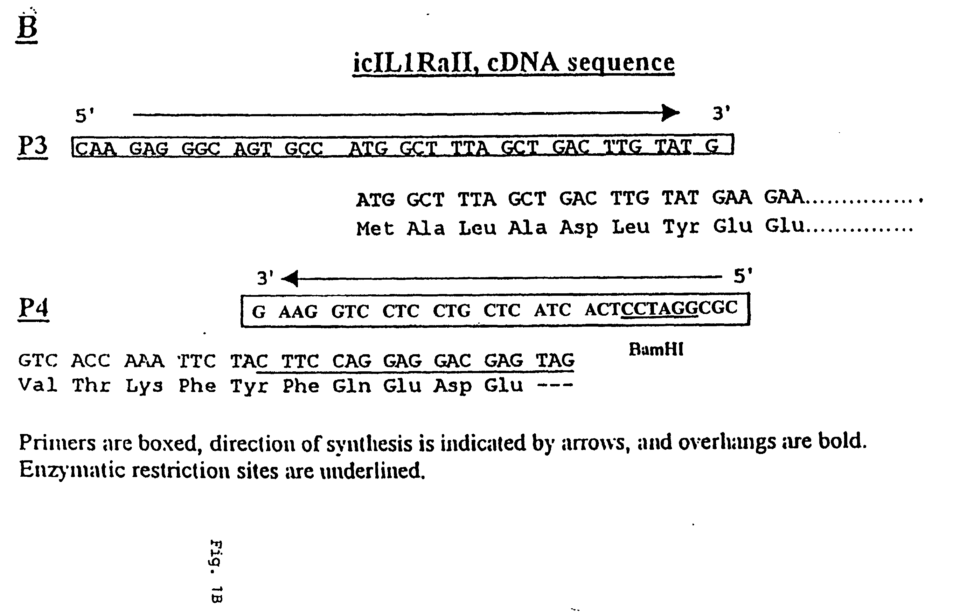

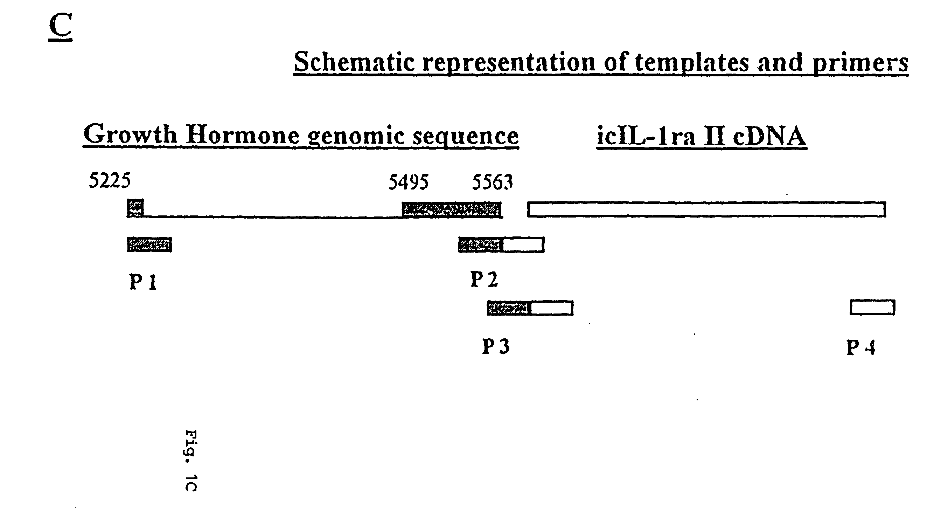

Figures 1A-1C show, in Fig. 1A, the genomic hGH signal peptide DNA sequence (SEQ ID NO:1), its amino acid sequence (SEQ ID NO:2), and the primers P1 and P2; in Fig. 1B, the beginning (SEQ ID NO:3) and the end (SEQ ID NO:5) of icIL-1ra type II cDNA, their amino acid sequences (SEQ ID NO:4 and NO:6), and primers P3 and P4 used for construction of the fusion constructs, and in Fig. 1C a schematic representation of templates and primers.
   **P1:** hGH-sp 5' primer, containing HindIII restriction site (SEQ ID NO:7).
   **P2:** hGH-sp 3' primer, containing 3'icIL-1ra-II sequence overhang (SEQ ID NO:8).
   **P3:** icIL-1ra-II 5' primer, containing 5' hGH-sp sequence overhang (SEQ ID NO:9).
   **P4:** icIL-1ra-II 3' primer, containing two stop codons and BamHI restriction site (SEQ ID NO:10).

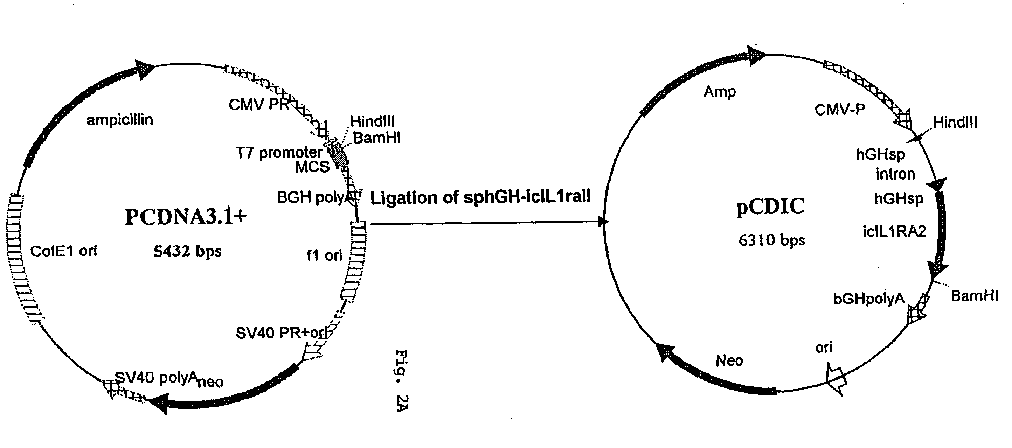

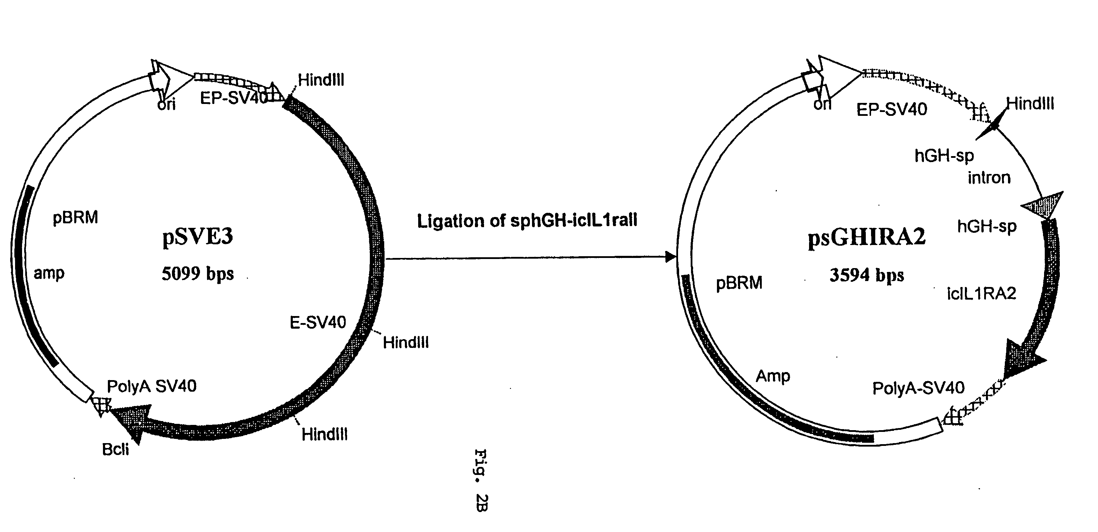

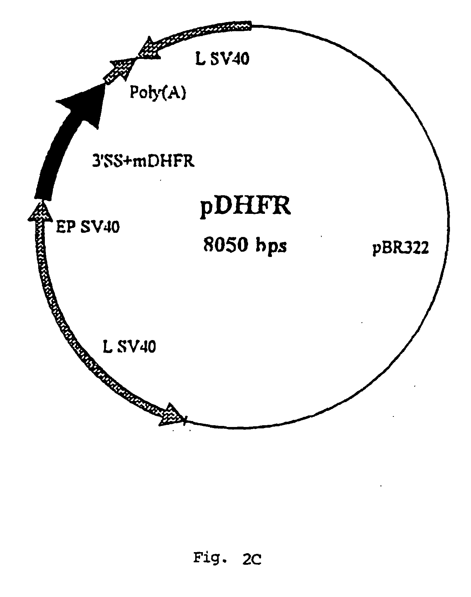

**Figures 2A-2C** describe, in Fig. 2A, the construction of the pCDIC and, in Fig. 2B, the construction of pSGHIRA2 DNA vectors used for expression of the icIL-1ra type II in mammalian cells. Fig. 2C is a scheme of pDHFR.

### Detailed Description of the Invention

The natural form of icIL-1ra type II is expressed intracellularly and is not secreted by the cells in which it is produced. However, in accordance with the present invention, this protein can be secreted in a mammalian recombinant production system by fusing the DNA encoding the protein to the DNA encoding the 26-amino acid signal peptide of the human growth hormone gene.

Prior to the present invention, it could not have been reasonably predicted whether or not the hGH signal peptide would drive the expression of icIL-1ra-II in a mammalian cell expression system in view of the fact that icIL-1ra-II is naturally expressed only intracellularly and is not secreted from the cell. In the known prior art, as represented by Pecceu et al (1991), the hGH signal peptide was used to express and secrete the mature form of IL-1β. However, the mature form of IL-1β is naturally secreted from the cells in which it is produced, although indirectly. A precursor protein is first produced which, after intracellular processing, is secreted from the cell. However, Pecceu discloses that when a recombinant vector containing only the DNA encoding the mature form of IL-1β, without any signal protein, is used, the protein is secreted from CHO cells. Thus, it could not be predicted with a reasonable degree of certainty that a protein such as icIL-1ra-II, which is only expressed intracellularly and is not naturally secreted from the cell, could be made to be secreted in large quantities in a recombinant mammalian expression system when fused to an hGH signal peptide.

The icIL-1ra-II protein produced in accordance with the present invention is glycosylated while the natural protein is non-glycosylated. Thus, the present invention further relates to the two novel glycosylated forms of icIL-1ra-II produced for the first time by means of the present invention. These are the glycosylated forms which have apparent molecular weight of approximately 27 kDa and 30 kDa as determined by Commassie blue staining of SDS-PAGE (15% acrylamide under reducing conditions). It could. not be predicted with a reasonable degree of certainty whether these novel glycosylated forms of icIL-1ra-II will retain the biological activity of natural icIL-1ra-II and will not be immunogenic when administered to humans. Experiments with these two novel glycosylated forms of icIL-1ra-II will establish that they are indeed biologically active and non-immunogenic when administered to humans.

Accordingly, the present invention is directed to a process for the recombinant expression of a protein having the amino acid sequence of natural icIL-1ra-II in a recombinant cell expression system through use of a vector which is a fusion of the signal peptide of the 26 amino acid signal peptide of hGH, fused in proper reading frame with the DNA encoding icIL-1ra-II. The process comprises producing an expression vector containing DNA encoding icIL-1ra-II, either in the form of cDNA or genomic DNA, fused in proper reading frame with DNA encoding the 26 amino acid hGH signal peptide. The expression vector is then inserted into an appropriate expression host, such as CHO cells. The transformed host cells are then cultured in a manner which causes the expression vector to express its encoded protein and the expressed and secreted icIL-1ra-II protein is then collected and purified from the culture medium.

The present invention is not intended to be limited by the specific examples presented herein. While CHO cells are used as the host cells, any other eukaryotic expression system, preferably mammalian expression system, may be used such as COS cells, yeast cells, insect cells, etc. Those of ordinary skill in the art are well aware of the techniques of creating expression vectors, inserting them into expression systems and selecting clones which express the desired protein, including amplification techniques.

As would be appreciated by those skilled in the art, the types of promoters used to control transcription of the icIL-ra-II proteins may be any of those which are functional in the host cells. Examples of promoters functional in mammalian cells include the SV40 early promoter, adenovirus major late promoter, herpes simplex (HSV) thymidine kinase promoter, rous sarcoma (RSV) LTR promoter, human cytomegalovirus (CMV)immediate early promoter, mouse mammary tumor virus (MMTV) LTR promoter, interferon-β promoter, heat shock protein 70 (hsp 70) promoter, as well as many others well known in the art. These promoters may be either constitutive or regulatable. All else being equal, constitutive promoters are preferred because an extra treatment step, such as temperature shift, addition of chemical agents or inducers, etc., is not required for expression from constitutive promoters.

The technical advance of the present invention lies in the confirmation that icIL-1ra-II can be secreted in such an expression system when using a signal peptide of a human secretory protein, preferably hGH. All of the other techniques involved are well known to those of ordinary skill in this art and can be practiced without undue experimentation using only the knowledge of the skill of the art available at the time of the present invention.

The present invention further is directed to the expression vector which contains the icIL-1ra-II DNA fused to the DNA encoding a signal protein of the human secretory protein hGH, and host cells. transfected with such an expression vector.

The present invention is further directed to the novel glycosylated forms of icIL-1ra-II produced in accordance with the present invention.

The invention further relates to methods for manufacturing a pharmaceutical composition for reducing the amount of IL-1 in patients having a condition involving the overexpression of IL-1, by the use of one of the novel glycosylated icIL-1ra-II proteins in accordance with the present invention. Appropriate therapeutic dosages for the reduction of IL-1 in patients having such a condition, can be readily empirically determined by those of ordinary skill in the art.

The glycosylated icIL-1ra-II proteins of the present invention may be administered by any means that achieves its intended purpose. For example, administration may be by a number of different parenteral routes including, but not limited to, subcutaneous, intravenous, intradermal, intramuscular, intraperitoneal, intracerebral, intranasal, oral, transdermal, or buccal routes. Parenteral administration can be bolus injection or by gradual perfusion over time.

It is understood that the dosage administered will be dependent upon the age, sex, health, and weight of the recipient, kind of concurrent treatment, if any, frequency of treatment, and the nature of the effect desired. The total dose required for each treatment may be administered by multiple doses or in a single dose. By "effective amount", it is meant a concentration of glycosylated icIL-1ra-II protein which is capable of reducing the amount of IL-1 in patients having a condition involving elevated levels of IL-1. Such concentrations can be routinely determined by those of skill in the art. It will also be appreciated by those of skill in the art that the dosage may be dependent on the stability of the administered protein. A less stable protein may require administration in multiple doses.

The invention also relates to pharmaceutical compositions comprising the glycosylated icIL-1ra-II protein of the present invention with a pharmaceutically acceptable excipient.

Preparations for parenteral administration include sterile aqueous or non-aqueous solutions, suspensions, and emulsions, which may contain auxiliary agents or excipients which are known in the art.

Pharmaceutical compositions comprising the glycosylated icIL-1ra-II protein according to the invention include all compositions wherein the protein is contained in an amount effective to achieve its intended purpose. In addition, the pharmaceutical compositions may contain suitable pharmaceutically acceptable carriers comprising excipients and auxiliaries which facilitate processing of the active compounds into preparations which can be used pharmaceutically. Suitable pharmaceutically acceptable vehicles are well known in the art and are described for example in Gennaro, Alfonso, Ed., Remington's Pharmaceutical Sciences, 18th Edition, Mack Publishing Co., Easton, PA (1990), a standard reference text in this field. Phannaceutically acceptable vehicles can be routinely selected in accordance with the mode of administration and the solubility and stability of the protein. For example, formulations for intravenous administration may include sterile aqueous solutions which may also contain buffers, diluents and other suitable additives.

While any excipients known for the administration of therapeutic proteins can be used in accordance with the present invention, excipients used for intravenous administration are preferred.

Suitable formulations for parenteral administration include aqueous solutions of the active compounds in water-soluble form, for example, water-soluble salts. In addition, suspension of the active compound as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions that may contain substances which increase the viscosity of the suspension include, for example, sodium carboxymethyl cellulose, sorbitol, and/or dextran. Optionally, the suspension may also contain stabilizers.

The vectors in accordance with the present invention can also be used for the manufacture of a pharmaceutical composition for use in gene therapy to cause appropriate human cells to express icIL-1ra-II *in vivo* in order to direct the IL-1 antagonizing effect of this protein directly at the desired site. The novelty of this process lies in the particular vector and the knowledge of the activity of the glycosylated protein produced thereby and not in the specific methods of gene therapy, including the methods of introducing an expressible vector directly into the cells of interest. These are within the skill of those of ordinary skill in this art at the time the present invention was made.

Non-limiting specific examples of the present invention follow.

### Example 1: Generation of hGH-sp-icIL-ra-II Fragment

The human growth hormone hGH signal peptide sequence was amplified by PCR using the pXGH5 vector, which encodes the full-length human growth hormone genomic sequence, as template (DeNoto et al, 1981). pXGH5 was used as a template for PCR using primers P1 (containing a HindIII restriction site and a Kozak sequence) and P2 (which has a 3' icIL-1ra-II sequence overhang, Fig. 1A). The icIL-1ra-II cDNA was amplified by PCR with primers P3 (which has a 5' hGH signal peptide sequence overhang) and P4 (which has two stop codons and a BamHI restriction site, Fig. 1B). These two PCR fragments were annealed together by their homologous regions and were further amplified by PCR using primers P1 and P4, to generate the hGH-icIL-1ra-II fragment (Fig. 1C).

### Example 2: Construction of pCDIC

hGH-sp-icIL-1ra-II fragment was digested with HindIII and BamHI and cloned into the HindIII-BamHI sites of pCDNA3.1 (+) (Invitrogen, San Diego, Fig. 2A) downstream of the CMV promoter. The resulting vector (pCDIC, Fig. 2A) was mapped by restriction analysis, and used to transfect COS cells.

### Example 3: Construction of pSGHIRA2

hGH-sp-icIL-1ra-II fragment was digested with HindIII and BamHI, and cloned into HindIII-BclI sites of pSVE3 (Fig. 2B; Hartman et al, 1982), downstream of the early SV-40 promoter. The resulting vector pSGHIRA2 (Fig. 2B) was used to transfect CHO DUKX (ATCC, CRL 9010) cells in co-transfection with the mouse DHFR containing vector pDHFR (Fig. 2C) as detailed below. The constructs were analyzed by restriction mapping and sequenced.

### Example 4: Expression Vector Carrying the Mouse DHFR Gene

Plasmid pDHFR (Fig. 2C) is composed of the complete pBR322 sequence, the SV40 early gene promoter, the 70 bp splicing region of the mouse γ2a gene fused to the mouse DHFR cDNA, followed by the SV40 early gene polyadenylation signal.

### Example 5: Transient Expression in COS Cells

pCDIC DNA was used for transfecting COS cells by means of the DEAE dextran method. Cells were seeded at approximately 3x10⁶/80 cm² flask and allowed to grow overnight. The next day all medium was aspirated from the flasks and 5 ml of transfection medium was added to the cells. Transfection medium contains 400 µg/ml DEAE dextran 100 µM Chloroquine, 2 µg/ml DNA, and 10% NuSerum in RPMI medium. After incubation for 3-4 hrs at 37°C the transfection medium was removed by aspiration and replaced with 5 ml of 10% DMSO in PBS for 2 minutes at room temperature. This solution was then aspirated and culture medium containing 10% FBS in RPMI added to the flasks. The cultures were incubated at 37°C for 24 hours, then the culture medium was changed to medium containing 2% serum. 24 hours later incubation temperature was reduced to 32°C, and culture supernatant samples were analyzed for the presence of the icIL-1ra type II in the culture supernatant by ELISA (see Example 7). 6-7 µg/1x10⁶ cells/run icIL-1ra type II were secreted from the transfected COS cells. Highest production levels were at days 4 to 8 following transfection. These results indicate that fusion of the hGH signal peptide to the intracellular form of IL-1-1ra type II, enables its secretion into the culture medium of the transfected cells.

### Example 6: Stable Expression in CHO Cells

CHO cells were cotransfected with pSGHIRA2 vector carrying the genes for icIL-1ra type II and with pDHFR carrying the gene for mouse DHFR (described in Fig. 2C), by means of the Lipofectamine transfection method.

Cells were seeded 1x10⁶/10 cm plate in F12 medium containing 10% FCS, and allowed to grow overnight. The cells were washed in F 12 medium, and 8 ml of the DNA-Lipofectamine mixture was added to the plates, which were then incubated for 4-5 hours at 37°C. At the end of the incubation period, 8 ml of F12 medium containing 20% FCS were added, and the plates were incubated for 24 hours at 37°C. The culture medium was then changed to fresh F12 medium containing 10% FCS. 72 hours following transfection cell cultures were seeded, either by limiting dilution, or by subculture at a 1:20 dilution, into selective medium depleted of Thymidine and Hypoxantine, containing 10% dialyzed FCS, and allowed to grow until single colonies could be picked and analyzed. Expression of icIL-1ra-II by eight stably integrated CHO clones is summarized in Table 1. The process lends itself to scale up by methods known in the art.

**Table 1**

| **Specific Productivity of icIL-1ra-II by CHO Clones** | | |
|---|---|---|
| Clone Number | Specific Productivity ng/10⁶ cells/day | |
| | Before MTX Amplification | After MTX Amplification (up to 400 nM MTX) |
| 1-33 | 118 | 273 |
| 2-56 | 91 | 220 |
| 1-64 | 122 | 186 |
| 1-84 | 194 | 245 |
| 2-2 | 92 | 524 |
| 2-66 | 124 | 283 |
| 2-73 | 82 | 218 |
| 2-88 | 120 | 442 |

### The MTX amplification was performed as follows:

Cells that grew in the absence of MTX were seeded to six T-flasks, in the presence of different MTX concentrations (e.g., 0, 2nM, 5nM, 10nM, 20nM, 50nM). About 10 days later, the cultures were observed microscopically and the cells were counted, in order to determine survival. The MTX concentration that allowed the survival of approximately 10% of the culture was selected for further propagation. The second round of amplification was performed in a similar manner, however the MTX concentrations were higher, starting from the MTX concentration that was selected at the first round. The cultures were again scored for survival, relative to the control MTX concentration of this round. The clones presented in Table 1 were amplified to the MTX concentrations shown in Table 2.

**Table 2**

| Clone | MTX First Round | MTX Second Round |
|---|---|---|
| 1-33 | 20nM | 300nM |
| 1-64 | 20nM | 400nM |
| 1-84 | 20nM | 100nM |
| 2-2 | 50nM | 200nM |
| 2-56 | 50nM | 100nM |
| 2-66 | 20nM | 100nM |
| 2-73 | 20nM | 100nM |
| 2-88 | 20nM | 100nM |

### Example 7: ELISA Test

Microtiter plates (Nunc) were coated with mouse anti IL-1ra antibody (purified ascitis IgG, MCA 1467, clone 1384, Serotec Ltd, Oxford, UK) 5 µg/ml in PBS (100 µl/well), for 3 hrs at 37°C, and stored 40°C. The plates were washed with PBS containing Tween 20 (0.05%, referred to herein as washing buffer) and blocked with the same solution containing 1% bovine serum albumin (BSA, referred to herein as blocking solution) for 1 hour at 37°C. Plates were then washed in washing buffer. The samples to be analyzed were diluted in the blocking solution, and added to the wells (100 µl/well) for 90 minutes at 37°C. The plates were then washed 6 times in washing buffer, followed by addition of biotinylated anti human IL-1ra antibody (100 µl/well of a 1:10,000 dilution, MCA 1466B, clone 1390m, Serotec, Oxford, UK). Plates were incubated for 1 hr at 37°C and washed with washing buffer. A horseradish peroxidase (HRP) streptavidin conjugate (1 mg/ml Sigma, Rehovot, Israel, 100 µl/well diluted 1:10,000 in blocking buffer) was then added to the plates, and incubated for 1.5 hours at 37°C. The plates were then washed in buffer and the substrate solution (*o*-phenylenediamine dihydrochloride, OPD, Sigma Rehovot, Israel, 100 µl/well) was added for 10 min. at 22°. The reaction was stopped by addition of 100 µl/well of 4N HCl. The plates were then read in an automated Elisa reader. A standard preparation of IL-1ra (Serotec, Oxford, UK, PHP080, 2-128 ng/ml) was used as reference for the IL-Ira concentration.

### Example 8: Affinity Chromatography of icIL-1ra Type II with Monoclonal Antibodies

Affinity chromatography of icIL-1ra type II was performed by binding anti-human IL-1ra antibodies (purified ascitis IgG, MCA 1467, clone 1384, Serotec Ltd, Oxford, UK) to CNBr activated Sepharose 4B (5 mg/ml resin, Pharmacia, Uppsala, Sweden). Culture supernatant from CHO cells, of clone 2-88 of the above-mentioned Example 6 was diafiltrated over a 100K membrane and then concentrated over a 10K membrane. Concentrated proteins were dialyzed against 0.1 M NaHCO₃, 150 mM NaCl pH 8.2. This procedure enriched the product concentration, reduced the volume of sample (100 fold) and removes major impurities. The yield of this step is about 85%. 30 ml of concentrated proteins were loaded on a 3.2 ml column, that had been equilibrated with 0.1 M sodium carbonate, 150 mM sodium chloride, pH 8.3, at a flow rate of 2 ml/min. icIL-1ra was eluted in 150 mM citric acid, 300 mM NaCl pH 2.7. Eluted fractions were immediately neutralized with 1M Tris pH 9.3. The fraction eluted resolved into two bands of apparent molecular weight of approximately 27 kDa and 30 kDa respectively, as determined by Commassie blue staining of SDS-PAGE (15% acrylamide under reducing conditions). The different molecular weights are presumably due to variations in glycosylation.

### Example 9: Western Blot

The affinity chromatography column eluate of above-mentioned Example 8 was concentrated by filtration on a 3K membrane (Miniset, Pall Filtron, Northborough, MA). Fractions were resolved on a 15% acrylamide SDS-PAGE gel under reducing conditions (Readygel BioRad, Hercules, CA) and electroblotted onto a nitrocellulose membrane (BRL, Life Technologies, MD). The blot was incubated in PBS containing 10% low fat milk, 0.1% Tween 20, overnight. The blot was then incubated with mouse anti human IL-1ra antibodies (purified ascitis IgG 1:5,000, MCA 1467, Serotec Ltd, Oxford, UK) for 2 hours at RT, then washed three times for 15 minutes in PBS containing 0.1% Tween 20, and further incubated with goat anti-mouse horseradish peroxidase-alkaline phosphatase (1:10,000 Sigma, Israel) for 1 hour at RT. The blot was then washed 3 times in PBS containing 0.1% Tween 20, followed by detection with enhanced luminescence (Amersham). Two protein bands of approximately 27 kDa and 30 kDa, respectively, corresponding to icIL-1ra, were identified.

### Example 10: Protein Sequence Analysis

The purified fraction from the immunoaffinity chromatography column of Example 8 was electroblotted in parallel, both on a PVDF membrane (Millipore, Bedford, MA), and on a nitrocellulose membrane for Western blotting analysis as described in the above-mentioned Example 9. The two bands stained by Coommassie blue were both recognized as IL-1ra by Western blot analysis. The purified fraction eluted from the affinity chromatography column, as well as the two bands excised from the Coomassie blue stained PVDF membrane, were subjected to protein sequence analysis by Edman degradation in the Procise™, 491HT microsequencer (Applied Biosystems, USA). Sequencing of the N-terminal amino acids, indicated that the purified fraction separated from the culture supernatant contained two forms of icIL-1ra. The amino acid sequence obtained, ALADLYEEGGGGGGE (SEQ ID NO:11), demonstrated that the secreted protein represented the mature icIL-1ra type II protein beginning at amino acid position +2 from the deduced start of translation of the gene (GENBANK, ID# X84348). An additional icIL-1ra form, beginning at amino acid +1 from the deduced start of translation of the icIL-1ra type II, was found as well.

Having now fully described this invention, it will be appreciated by those skilled in the art that the same can be performed within a wide range of equivalent parameters, concentrations, and conditions without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

Reference to known method steps, conventional method steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### REFERENCES

Bertini et al, "Inhibitory effect of recombinant intracellular interleukin 1 receptor antagonist on endothelial cell activation", Cytokine 4(1):44-47 (1992)
Bjorkdahl et al, "Gene transfer of a hybrid interleukin-1 beta gene to B 16 mouse melanoma recruits leucocyte subsets and reduces tumour growth in vivo", Cancer Immunol Immunother 44(5):273-81 (1997)
Carter et al, "Purification, cloning, expression and biological characterization of an interleukin-1 receptor antagonist protein", Nature 344(6267):633-638 (1990)
Colotta et al, "Interleukin-1 type II receptor: a decoy target for IL-1 that is regulated by IL-4", Science 261(5120):472-475 (1993)
Colotta F et al, "The type II 'decoy' receptor: a novel regulatory pathway for interleukin 1, Immunol Today 15(12):562-566 (1994)
DeNoto et al, "GoHM Human growth hormone DNA sequence and mRNA structure: possible alternative splicing", Nucleic Acids Res 9(15):3719-3730 (1981)
Dinarello, CA "Interleukin-1 and interleukin-1 antagonism", Blood 77(8):1627-1652 (1991)
Dinarello, CA "Biologic basis for interleukin-1 in disease", Blood 87(6):2095-2147 (1996)
Eisenberg et al, "Primary structure and functional expression from complementary DNA of a human interleukin-1 receptor antagonist", Nature 343(6256):341-346 (1990)
Gennaro, Alfonso, Ed., Remington's Pharmaceutical Sciences, 18th Edition 1990, Mack Publishing Co., Easton, PA,
Hannum et al, "Interleukin-1 receptor antagonist activity of a human interleukin-1 inhibitor", Nature 343(6256):336-340 (1990)
Hartman et al, "Human influenza virus hemagglutinin is expressed in monkey cells using simian virus 40 vectors", Proc Natl Acad Sci USA, 79 (2):233-237 (1982)
Haskill et al, "cDNA cloning of an intracellular form of the human interleukin 1 receptor antagonist associated with epithelium, Proc Natl Acad Sci USA 88(9):3681-3685 (1991)
Komada et al, "Protective effect of transfection with secretable superoxide dismutase (SOD) (a signal sequence-SOD fusion protein coding cDNA) expression vector on superoxide anion-induced cytotoxicity in vitro", Biol Pharm Bull 20(5):530-6 (1997)
Muzio et al, "Cloning and characterization of a new isoform of the interleukin 1 receptor antagonist," J Exp Med 182(2):623-628 (1995)
Pecceu et al, "Human interleukin 1β fused to the human growth hormone signal peptide is N-glycosylated and secreted by Chinese hamster ovary cells", Gene 97(2):253-258 (1991)
Selden et al, "Human growth hormone as a reporter gene in regulation studies employing transient gene expression", Mol Cell Biol 6(9):3173-3179 (1986)
Sims et al, "Interleukin 1 signaling occurs exclusively via the type I receptor", Proc Natl Acad Sci USA, 90(13):6155-6159 (1993)

## Claims

1. An expression vector, comprising a DNA segment encoding the human growth hormone signal peptide joined to a DNA segment encoding intracellular IL-1 receptor antagonist type II (icIL-1ra-II) and operably linked to a promoter sequence, wherein said icIL-1ra-II is expressed from said promoter sequence and translated with said signal peptide fused in frame to icIL-1ra-II.

2. A host cell transformed with the expression vector of claim 1.

3. The expression vector in accordance with claim 1 for the use in gene therapy.

4. The expression vector in accordance with claim 3 for introduction into appropriate endogenous human cells at the desired site to produce transformed cells which will express icIL-1ra-II at the desired rate.

5. A method for producing a recombinant iclL-1ra-II comprising the steps of :
culturing a host cell according to claim 2 to express and produce a recombinant glycosylated icIL-1ra-II;
recovering the produced recombinant glycosylated icIL-1ra-II.

6. A glycosylated icIL-1ra-II produced by a method according to claim 5.

7. The glycosylated icIL-1ra-II according to claim 6 having an apparent molecular weight of about 27 kDa on SDS-PAGE under reducing conditions with 15% acrylamide.

8. The glycosylated iclL-1ra-II according to claim 6 having an apparent molecular weight of about 30 kDa on SDS-PAGE under reducing conditions with 15% acrylamide.

9. A pharmaceutical composition, comprising the glycosylated icIL-1ra-II according to claim 6 in a therapeutically effective amount and a pharmaceutically acceptable excipient.

## Patentansprüche

1. Expressionsvektor, umfassend ein DNA-Segment kodierend das Signalpeptid des menschlichen Wachstumshormons verbunden mit einem DNA-Segment kodierend den intrazellulären IL-1-Rezeptorantagonist Typ II (icIL-1ra-II) und operativ verknüpft mit einer Promotorsequenz, wobei der icIL-1ra-II von der Promotorsequenz exprimiert wird und mit der Signalsequenz, die im Leseraster mit icIL-1ra-II fusioniert ist, translatiert wird.

2. Wirtszelle transformiert mit dem Expressionsvektor nach Anspruch 1.

3. Expressionsvektor nach Anspruch 1 zur Verwendung in der Gentherapie.

4. Expressionsvektor nach Anspruch 3 zum Einführen in geeignete endogene menschliche Zellen an einer gewünschten Stelle, um transformierte Zellen herzustellen, die icIL-1ra-II mit der gewünschten Rate exprimieren.

5. Verfahren zur Herstellung eines rekombinanten icIL-1ra-II umfassend die Schritte:
Kultivieren einer Wirtszelle nach Anspruch 2, um einen rekombinanten glykosilierten icIL-1ra-II zu exprimieren und herzustellen, Gewinnen des hergestellten rekombinanten glykosilierten icIL-1ra-II.

6. Glykosilierter icIL-1ra-II hergestellt durch ein Verfahren nach Anspruch 5.

7. Glykosilierter icIL-1ra-II nach Anspruch 6, der ein scheinbares Molekulargewicht von etwa 27 kDA auf SDS-PAGE unter reduzierenden Bedingungen mit 15 % Acrylamid hat.

8. Glykosilierter icIL-1ra-II nach Anspruch 6, der ein scheinbares Molekulargewicht von etwa 30 kDA auf SDS-PAGE unter reduzierenden Bedingungen mit 15 % Acrylamid hat.

9. Pharmazeutische Zusammensetzung, umfassend den glykosilierten icIL-1ra-II nach Anspruch 6 in einer therapeutisch wirksamen Menge und einen pharmazeutisch akzeptablen Träger.

## Revendications

1. Vecteur d'expression, comprenant un segment d'ADN codant pour le peptide signal de l'hormone de croissance humaine, joint à un segment d'ADN codant pour un antagoniste du récepteur intracellulaire IL-1 de type II (icIL-1ra-II) et lié de manière fonctionnelle à une séquence promoteur, ledit icIL-1ra-II étant exprimé à partir de ladite séquence promoteur et traduit avec ledit peptide signal fusionné en phase avec icIL-1ra-IL.

2. Cellule hôte transformée avec le vecteur d'expression de la revendication 1.

3. Vecteur d'expression selon la revendication 1 utilisé dans la thérapie génique.

4. Vecteur d'expression selon la revendication 3 à introduire dans des cellules endogènes humaines appropriées au site souhaité pour produire des cellules transformées qui exprimeront icIL-1ra-II au niveau souhaité.

5. Procédé de production d'un icIL-1ra-II comprenant les étapes de : culture d'une cellule hôte selon la revendication 2 pour exprimer et produire un icIL-1ra-II glycosylé recombinant ; et récupération du icIL-1ra-II glycosylé recombinant produit.

6. icIL-1ra-II glycosylé produit par un procédé selon la revendication 5.

7. icIL-1ra-II glycosylé selon la revendication 6 ayant un poids moléculaire apparent d'environ 27 kDa sur SDS-PAGE dans des conditions réductrices avec 15 % d'acrylamide.

8. icIL-1ra-II glycosylé selon la revendication 6 ayant un poids moléculaire apparent d'environ 30 kDa sur SDS-PAGE dans des conditions réductrices avec 15 % d'acrylamide.

9. Composition pharmaceutique comprenant le icIL-1ra-II glycosylé selon la revendication 6 en une quantité thérapeutiquement efficace et un excipient pharmaceutiquement acceptable.
